# EUROPEAN PATENT SPECIFICATION

(11) **EP 2 132 175 B1**
(45) Date of publication and mention of the grant of the patent: **02.05.2012**
(21) Application number: 07866264.0
(22) Date of filing: 24.12.2007
(51) Int. Cl.: C07D 211/52, A61K 31/445, A61P 1/12

(54) **PROCESS FOR THE PREPARATION OF LOPERAMIDE**
VERFAHREN ZUR HERSTELLUNG VON LOPERAMID
PROCÈDE POUR LA PRÉPARATION DE LOPERAMIDE

(30) Priority: 28.12.2006 ES 200700030
(43) Date of publication of application: 16.12.2009
(60) Divisional application: 11159364.6
(73) Proprietor: Institut Univ. de Ciència i Tecnologia, S.A., 08100 Mollet del Vallès Barcelona (ES)
(72) Inventor: ESTÉVEZ COMPANY, Carles, 08100 Mollet del Vallès (ES); BAYARRI FERRER, Navidad, 08100 Mollet del Vallés (ES); CASTELLS BOLIART, Josep, 08100 Mollet del Vallès (ES); ECHEVERRÍA BEISTEGUI, Begoña, 08100 Mollet del Vallès (ES)
(74) Representative: Ponti Sales, Adelaida
(86) International application number: PCT/EP2007/011420
(87) International publication number: WO 2008/080601

(56) References cited:
- WO-A-99/03457
- US-A- 3 884 916
- CHEN ZHENGMING ET AL: "Design and synthesis of 4-phenyl piperidine compounds targeting the mu receptor." BIOORGANIC & MEDICINAL CHEMISTRY LETTERS 1 NOV 2004, vol. 14, no. 21, 1 November 2004 (2004-11-01), pages 5275-5279, XP002479423 ISSN: 0960-894X

## Description

The present invention relates to the field of inert solvents useful for carrying out chemical reactions and, more particularly, to the preparation of loperamide using glycerol formal as a solvent.

### BACKGROUND OF THE INVENTION

An appropriate selection of the solvent in the different reaction or purification steps may enhance the yield of the active ingredient, or determine characteristics such as crystal form, purity, and solubility. Therefore, the solvent is a critical parameter in the preparation of drug substances. Moreover, the level of residual solvents in the end product must be reduced to an acceptable amount in order to meet product specifications according to safety requirements. Guideline for Residual Solvents (CPMP/ICH/283/95) and subsequent revisions establish a classification and the limits of residual solvents based on safety purposes.

Known hazardous solvents should be avoided in the production of both the pharmaceutical active ingredient and the final pharmaceutical product because they may cause unacceptable toxicities or deleterious environmental effects. Examples of these solvents are carbon tetrachloride, 1,2-dichloroethane, 1,1-dichloroethane and 1,1,1-trichloroethane.

Other solvents associated with a lower toxicity level should be limited in order to protect patients from potential adverse effects. Examples of these solvents are xylene, toluene, methanol, hexane, N,N-dimethylformamide (DMF), N,N-dimethylacetamide (DMA), dichloromethane, chloroform and acetonitrile.

Solvents that may be regarded as less toxic are acetone, ethanol, isopropyl alcohol (IPA), ethyl acetate, dimethylsulfoxide (DMSO), ethyl ether, methylethylketone (MEK), methylisobutylketone (MIBK), propanol and tetrahydrofuran (THF). These solvents should be employed when their use can be justified by the process or product characteristics.

Although, due to their lower toxicity, the solvents from the aforesaid third class are the preferred ones, they should be still used according to valid regulations that establish parameters so as to minimize their impact on both human health and environment. Thus, if MIBK is taken as an example, since it is one of the most widely used solvent in the chemical industry due, among other reasons, to its ability to solubilize organic compounds and to the fact that it can be easily removed by evaporation, there may be some risks associated with its use. The intrinsic hazard of MIBK is essentially evidenced by its physical properties (bp: 117-118 °C; vP: 15 mmHg (20 °C)) and toxicological data (LD₅₀ (oral-rats): 2.1 g/kg), in such a way that European legislation has established indicative environmental and biological limits. Consequently, Spanish legislation has included MIBK in the general list of environmental limit values of professional exposure and established a daily exposure value (ELV-DE) of 83 mg/m³ and a short exposure value (ELV-SE) of 208 mg/m³. MIBK exhibits the following R-phrases ("Risk Phrases"): 11-20-36/37-66 ("Highly flammable. Harmful by inhalation. Irritating to eyes and to respiratory system. Repeated exposure may cause skin dryness or cracking"). From the viewpoint of safety, MIBK exhibits the following S-phrases: 9-16-29 ("Keep container in a well-ventilated place. Keep away from sources of ignition - No smoking. Do not empty into drains"). The hazard codes of the National Fire Protection Association (NFPA) assign the score of 3 the flammability of MIBK and the score of 2 its hazard to human health.

The primary objective of the development of a pharmaceutical active ingredient is to find one or various synthetic routes which allow to obtain the concerned compounds with an acceptable yield. This implies that suitable reaction conditions, including solvents, should be used in order to achieve such objective at laboratory scale. Moreover, other additional aspects (e.g., solvents to be limited so as to meet drug product, safety, and environmental requirements) should be taken into account in a subsequent stage of process optimization and, specially, in its industrial scale-up.

Thus, in general, it is of a great interest to the pharmaceutical industry the identification and use of alternative solvents in order to minimize the problems associated with solvents that are usually employed in known preparation processes of active ingredients.

US 3,884,916 and WO 99/03457 describe processes for the preparation of loperamide in 4-methyl-2-pentanone, where the resulting product is purified through recrystallisation in toluene and acetone. Zhengming Chen et al. describe processes for the preparation of loperamide in DMF (*Bioorg. Med. Chem. Lett.,* **2004**, *14*, 5275-5279).

### DESCRIPTION OF THE INVENTION

Surprisingly, it has been found that glycerol formal allows to carry out reactions for obtaining active pharmaceutical ingredients under highly safe and non-contaminant conditions. Thus, the end product, in addition to fully satisfy the safety requirements established by current regulations due to its low toxicity, shows several additional advantages.

Glycerol formal is a mixture of two isomers, 4-hydroxymethyl-1,3-dioxolane and 5-hydroxy-1,3-dioxane. These two isomers are present in constant proportion of 40% and 60%, respectively. Glycerol formal is very stable under both neutral and basic conditions and is inert toward the solubilized products which are compatible with the alcohol and acetal functions. In a strong acid medium, glycerol formal may be decomposed into formaldehyde and glycerol. In order to avoid its decomposition, the medium pH should preferably be higher than 4. Glycerol formal is completely miscible in ether, acetone, essential oils, alcohols and water. In addition, it is practically odourless, moderately biodegradable and presents a very low toxicity. It is known the use of glycerol formal in the state of the art, such as in the field of casting industry and in manufacturing household products, printing inks and paints. In the pharmaceutical and veterinary field, glycerol formal is employed as an excipient or vehicle in the formulation of products such as injectable solutions. However, nothing related to the use of glycerol formal as an inert solvent in chemical reactions for the preparation or purification of drug products is mentioned or suggested in the state of the art.

Table 1 shows the physicochemical properties of MIBK as compared with those of glycerol formal. The data for MIBK were obtained from the International Chemical Safety Card published by "Instituto Nacional de Seguridad e Higiene en el Trabajo" (National Institute for Occupational Safety and Hygiene). The data for glycerol formal were obtained from the most recent manufacturer's standard safety data sheet.

It can be seen that the boiling point and flash point of glycerol formal are approximately 80°C higher than those of MIBK and that the vapour pressure of glycerol formal is lower than that of MIBK, all of which promotes a lower exposure risk. Moreover, glycerol formal presents a better toxicological profile as evidenced by the absence of R-phrases and environmental limit values, as well as a five times higher lethal dose in rats (ingestion). The less-flammability of glycerol formal, as defined by the hazard codes of the NFPA, offers some advantages to the safety of its manipulation in a chemical plant.

**Table 1. Physicochemical properties of MIBK and Glycerol Formal**

| **PROPERTY** | **MIBK** | **GLYCEROL FORMAL** |
|---|---|---|
| **Colour** | Colourless liquid | Colourless liquid |
| **Odour** | Characteristic odour | Odourless liquid |
| **Boiling point (°C)** | 117-118 | 192-193 |
| **Flash point (°C)** | 18 | 98 |
| **Self-flash point (°C)** | 449 | >400 |
| **Explosivity limits (% v/v)** | 1.7 - 7.6 | Unavailable |
| **Vapour pressure (mmHg)** | 15 (exp., 20°C) | 0.13 (calc., 25°C) |
| **Density (25°C, g/mL)):** | 0.801 | 1.203 |
| **Solubility in H₂O (g/L)** | 20 | Miscible in all proportions |
| **LD₅₀(oral-rats, g/kg)** | 2.1 | 10 |
| **ELV-DE** | 83 mg/m³ 20 ppm | Unavailable |
| **ELV-SE** | 208 mg/m³ 50 ppm | Unavailable |
| **R-Phrases** | 11-20-36/37-66 | None |
| **S-Phrases** | 9-16-29 | None |
| **NFPA Codes** | Health: 2 | Health: 1 |
| | Flammability: 3 | Flammability: 1 |
| | Reactivity: 0 | Reactivity: 0 |

Therefore, an aspect of the present invention concerns a process for the preparation of the active pharmaceutical ingredient, loperamide or an acid addition salt thereof, wherein the reaction for obtaining said active ingredient is carried out in glycerol formal as an inert solvent, at a reaction pH higher than 4. It is understood as "inert solvent" that glycerol formal acts only as a solvent without taking part in the reaction as a reagent, without being chemically incompatible with any component of the reaction mixture and without being decomposed under the reaction conditions.

It should be emphasized among the advantages of the use of glycerol formal that it enables to carry out the preparation of active pharmaceutical ingredients under safer, non-contaminant conditions and with high yields. Thus, due to the fact that glycerol formal has a high boiling point, reactions can be carried out at high temperatures, what allows to accelerate or even optimize such reactions which, at lower temperatures, almost do not progress. Notwithstanding that, the use of glycerol formal was found to allow reactions to be carried out comparatively faster than in other solvents even at lower temperatures. Likewise, its high flammability point enables to operate safely at industrial levels and to carry out operations such as filtration at a temperature higher than room temperature or to handle the solvent or the wet products from the solvent with no flammability hazard.

In a preferred embodiment, the preparation of the active pharmaceutical ingredient is performed by a nucleophilic substitution reaction. Preferably, the nucleophilic substitution reaction is of S_{N}2 type.

In another preferred embodiment, the process for preparation of loperamide, or an acid addition salt thereof, comprises the reaction of a suitable salt of dimethyl(tetrahydro-3,3-diphenyl-2-furyliden)ammonium (1), preferably a halide and more preferably a bromide, with 4,4-chlorophenyl-4-hydroxypiperidine (2) or an acid addition salt thereof, as for example hydrochloride.

Scheme 1 depicts the process for the preparation of loperamide according to the present invention.

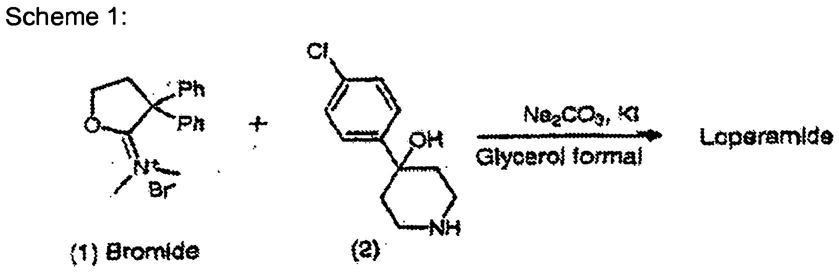

As in the course of the reaction an acid equivalent is released, for example hydrobromic acid when the bromide of ammonium derivative is used, the reaction is preferably carried out in the presence of a base, which will be combined with the acid released in the course of the reaction, in an amount from 1 to 1.2 equivalents in relation to 4,4-chlorophenyl-4-hydroxypiperidine. Examples of suitable bases are trialkylamines, heterocyclic tertiary amines and bases from alkaline or alkaline earth metals, such as hydroxides and alkaline or alkaline earthy metal carbonates, such as sodium hydroxide, potassium hydroxide, sodium carbonate and potassium carbonate. Preferably, the base is an alkaline or alkaline earth metal carbonate, even more preferably sodium carbonate. Likewise, it is convenient to add a catalytic amount of potassium iodide.

In a more preferred embodiment, the reaction for obtaining loperamide is carried out at a temperature ranging from 40 to 70°C, preferably from 55 to 65°C. In an another preferred embodiment, glycerol formal is present in an amount comprised between 3 and 10 ml per each gram of 4,4-chorophenyl-4-hydroxypiperidine. More preferably the amount of glycerol formal is comprised between 3 and 5 ml, which is advantageous since productivity increases on working at high concentrations.

One of the advantages of employing glycerol formal in the preparation of loperamide is that the reaction can be carried out in a considerably faster way as compared with other solvents even at lower temperatures. Particularly, reaction times lower than 3 h can be reached.

Other of the advantages of employing glycerol formal in the process for the preparation of loperamide according to the present invention is that the active ingredient crude can be easily separated from the solid salts obtained by centrifugation. Particularly, a centrifugation at 40°C enables to isolate the product with a good yield and a good purity. Increasing the usual temperature interval to which loperamide can be filtered is especially advantageous to the purification of certain impurities that are not soluble at a lower temperature, and there is no need of increasing the dilution.

In addition, the use of glycerol formal enables to perform the subsequent purification of loperamide without the utilisation of certain polar organic solvents, such as toluene and acetone which are used in the classical process described in the patent US 3,884,916.

Table 2 shows the values of different reaction parameters for the preparation of loperamide according to the classical process as compared with those corresponding to the process of the present invention.

**Table 2**

| | **Classical synthesis** | **Alternative synthesis** |
|---|---|---|
| **Solvent** | MIBK | Glycerol formal |
| **Reagents** | | |
| **(1) bromide** | 1 eq | 1 eq |
| **(2)** | 1.10 eq | 1.10 eq |
| **Na₂CO₃** | 2.60 eq | 1.10 eq |
| **Time** | Distillation + 15h | 2h |
| **Temperature** | 120 °C | 60 °C |
| **Purification** | IPA(HCl)/Toluene/ Acetone | IPA(HCl)/H₂O |

Despite an 80% yield obtained by the classical process versus a 67% yield obtained by the process of the present invention, the latter exhibits the advantage of requiring fewer base equivalents and being much more inexpensive because of significantly decreased reaction time and temperature.

Throughout the description and claims the word "comprise" and variations thereof are not intended to exclude other technical features, additives, components, or steps. Additional objects, advantages and features of the invention will become apparent to those skilled in the art upon examination of the description or may be learned by practice of the invention. The following examples and drawings are provided by way of illustration, and is not intended to be limiting of the present invention.

### EXAMPLES

### Example 1: Preparation of 4-[4-(4-diphenyl)-4-hydroxypiperidino]-N,N-dimethyl-2,2-diphenylbutyramide hydrochloride (loperamide)

0.111 g (5.2·10-4 mol, 1 eq) of 4,4-chlorophenyl-4-hydroxypiperidine, 0.062 g (5.8·10-4 mol, 1.11 eq) of sodium carbonate, 0.0009 g (0.24% by weight) of potassium iodide were weighed and dissolved in 0.5 mL of glycerol formal. The resulting mixture was stirred for 15 minutes. Afterwards 0.2089 g (6.03·10-4 mol, 1.15 eq) of N,N-dimethyl-(3,3-diphenyltetrahydro-2-furyliden)ammonium bromide were added and heated at 60°C. After 2 hours, the reaction mixture was left to cool to room temperature. The crude product was centrifuged at 18000 rpm for 30 minutes at 40°C. The two phases were separated. To the supernatant 0.65 mL of isopropanol saturated with hydrochloric acid (7.8·10-4 mol of HCl, 1.5 eq) was added by stirring for 20 minutes. Then, 1 mL of H₂O was added by stirring for 15 minutes. After this time, it was centrifuged at 9000 rpm for 1 hour at 21 °C and the supernatant was removed. It was washed with water. The obtained solid was decanted and dried under vacuum. The title compound was obtained as a white solid. Yield: 67%. Rf (AcNH₄/Dioxane/MeOH; 20/40/40) =0.86. ¹H NMR (300 MHz, CDCl₃) δ 11.7 (1 H, s, OH), 7.3-7.5 (14 H, m, Ar), 3 (1 H, s, CH₃), 2.36 (2H, t, CH₂, J=6 MHz), 2.25 (2H, t, CH₂, J=6 MHz), 14.94 (4H, t, J=6 MHz).

## Claims

1. Process for the preparation of loperamide or an acid addition salt thereof, **characterized in that** the reaction for obtaining said loperamide or acid addition salt thereof is carried out in glycerol formal as an inert solvent at a reaction pH higher than 4.

2. Process according to claim 1, comprising the reaction of dimethyl(tetrahydro-3,3-diphenyl-2-furylidene)ammonium halide with 4,4-chlorophenyl-4-hydroxypiperidine or an acid addition salt thereof.

3. Process according to claim 2, wherein said dimethyl(tetrahydro-3,3-diphenyl-2-furylidene)ammonium halide is dimethyl(tetrahydro-3,3-diphenyl-2-furylidene)ammonium bromide.

4. Process according any of the preceding claims, wherein the reaction is carried out in the presence of a base in an amount comprised between 1 and 1.2 equivalents in relation to 4,4-chlorophenyl-4-hydroxypiperidine.

5. Process according to any one of the preceding claims, wherein the reaction is carried out at a temperature comprised between 40 and 70°C.

6. Process according to claim 5, wherein the temperature is comprised between 55 and 65°C.

7. Process according to any one of the preceding claims, wherein glycerol formal is present in an amount comprised between 3 and 10 ml per each gram of 4,4-chlorophenyl-4-hydroxypiperidine.

## Patentansprüche

1. Verfahren zur Herstellung von Loperamid oder einem Säureadditionssalz hiervon, **dadurch gekennzeichnet, dass** die Reaktion, um Loperamid oder das Säureadditionssalz hiervon zu erhalten, in Glycerinformal als inertem Lösungsmittel bei einem Reaktions-pH von über 4 durchgeführt wird.

2. Verfahren nach Anspruch 1, umfassend die Reaktion von Dimethyl(tetrahydro-3,3-diphenyl-2-furyliden)ammoniumhalogenid mit 4,4-Chlorphenyl-4-hydroxypiperidin oder einem Säureadditionssalz hiervon.

3. Verfahren nach Anspruch 2, wobei das Dimethyl(tetrahydro-3,3-diphenyl-2-furyliden)ammoniumhalogenid Dimethyl(tetrahydro-3,3-diphenyl-2-furylidene)ammoniumbromid ist.

4. Verfahren nach einem der vorhergehenden Ansprüche, wobei die Reaktion in Gegenwart einer Base in einer Menge durchgeführt wird, umfassend 1 bis 1,2 Äquivalente in Bezug auf 4,4-Chlorphenyl-4-hydroxypiperidin.

5. Verfahren nach einem der vorhergehenden Ansprüche, wobei die Reaktion bei einer Temperatur, umfassend 40 bis 70°C, durchgeführt wird.

6. Verfahren nach Anspruch 5, wobei die Temperatur 55 bis 65°C umfasst.

7. Verfahren nach einem der vorhergehenden Ansprüche, wobei das Glycerinformal in einer Menge, umfassend 3 bis 10 ml pro jedem Gramm 4,4Chlorphenyl-4-hydroxypiperidin, vorliegt.

## Revendications

1. Procédé destiné à la préparation de lopéramide ou d'un sel d'addition d'acide de celui-ci, **caractérisé en ce que** la réaction pour obtenir ledit lopéramide ou sel d'addition d'acide de celui-ci est réalisée dans du glycérol formal en tant qu'un solvant inerte à un pH réactionnel supérieur à 4.

2. Procédé selon la revendication 1, comprenant la réaction d'un halogénure de diméthyl(tétrahydro-3,3-diphényl-2-furylidène) ammonium avec la 4,4-chlorophényl-4-hydroxypipéridine ou un sel d'addition d'acide de cette dernière.

3. Procédé selon la revendication 2, dans lequel ledit halogénure de diméthyl(tétrahydro-3,3-diphényl-2-furylidène) ammonium est le bromure de diméthyl(tétrahydro-3,3-diphényl-2-furylidène) ammonium.

4. Procédé selon l'une quelconque des revendications précédentes, dans lequel la réaction est réalisée en présence d'une base dans une quantité comprise entre 1 et 1,2 équivalents par rapport à la 4,4-chlorophényl-4-hydroxypipéridine.

5. Procédé selon l'une quelconque des revendications précédentes, dans lequel la réaction est réalisée à une température comprise entre 40° C et 70° C.

6. Procédé selon la revendication 5, dans lequel la température est comprise entre 55° C et 65° C.

7. Procédé selon l'une quelconque des revendications précédentes, dans lequel le glycérol formal est présent dans une quantité comprise entre 3 et 10 ml pour chaque gramme de 4,4-chlorophényl-4-hydroxypipéridine.
